# FASCICULE DE BREVET EUROPEEN

(11) **EP 2 908 790 B1**
(45) Date de publication et mention de la délivrance du brevet: **19.04.2017**
(21) Numéro de dépôt: 13789872.2
(22) Date de dépôt: 21.10.2013
(51) Int. Cl.: A61F 11/14, A61F 11/12

(54) **DISPOSITIF ET PROCÉDÉ DE PROTECTION AUDITIVE PERSONNELLE D'UN UTILISATEUR**
VORRICHTUNG UND VERFAHREN FÜR PERSONALISIERTEN GEHÖRSCHUTZ EINES BENUTZERS
DEVICE AND METHOD FOR THE PERSONAL AUDITORY PROTECTION OF A USER

(30) Priorité: 19.10.2012 FR 1260001
(43) Date de publication de la demande: 26.08.2015
(73) Titulaire: Earsonics, 34830 Clapiers (FR)
(72) Inventeur: LOPEZ, Franck, F-34830 Clapiers (FR); CHRYSOCHOOS, Thomas, F-34830 Clapiers (FR)
(74) Mandataire: Cornuejols, Georges
(86) Numéro de dépôt international: PCT/FR2013/052512
(87) Numéro de publication internationale: WO 2014/060711

(56) Documents cités:
- WO-A1-2011/050401
- US-A1- 2011 209 273
- US-B1- 6 456 199
- US-B1- 7 039 195

## Description

### DOMAINE TECHNIQUE DE L'INVENTION

La présente invention vise un dispositif de protection auditive personnelle d'un utilisateur. Elle s'applique notamment à la protection des travailleurs en milieu industriel et des ouvriers sur chantier.

### ETAT DE LA TECHNIQUE

Le bruit est reconnu comme une cause de maladies professionnelles (depuis 1963, en France, sur le tableau n°42 des maladies professionnelles relatif à la surdité provoquée par les bruits lésionnels). Dans le cadre de sites industriels ou de bâtiments ou ouvrages d'art en construction, les niveaux sonores varient grandement d'un instant à l'autre en raison de l'utilisation parfois ponctuelle, et imprévisible pour les ouvriers, de machines très bruyantes. Les protecteurs auditifs utilisés dans ces milieux possèdent de grands indices d'atténuation sonore, à tel point qu'il est difficile pour deux personnes équipées de tels protecteurs de s'entendre l'une l'autre. La nécessité de communiquer lors de l'exécution de certaines tâches industrielles pousse les ouvriers à ne pas porter à tout instant les protecteurs auditifs, parfois aux dépens de leur santé. De plus, les grandes variations de puissances sonores émises dans ces environnements, doublées d'une absence de douleur immédiate en cas de présence de certains bruits excédant le seuil de dangerosité, ne permettent pas à ces personnes de savoir quand porter leurs protecteurs auditifs.

Certains systèmes actuels avertissent les travailleurs de la nécessité de port de protecteurs auditifs. Ces systèmes affichent à différents emplacements sur les lieux d'opération, des messages indépendants du niveau sonore ambiant. L'inconvénient de ces systèmes est leur approche statique. Les travailleurs prennent l'habitude, pour se parler lorsque l'ambiance n'est pas très sonore, de ne pas porter de protection auditive puis, en s'habituant au bruit ambiant, de ne jamais porter cette protection.

De plus, ces systèmes ne permettent pas à un employeur de suivre l'utilisation des protecteurs auditifs par ses collaborateurs.

On connaît des systèmes de protection auditive réalisés sur mesure, par exemple pour des artistes, des musiciens, des gens du spectacle ou certains travailleurs. Ces systèmes sont très onéreux puisqu'ils sont réalisés après prise de mesures ou moulage sur l'oreille interne de l'utilisateur.

Les dispositifs décrits dans les documents US 6 456 199, WO 2011/050401 et US 2011/209273 présentent tout ou partie de ces inconvénients.

De plus, aucun des systèmes connus dans l'art antérieur, notamment dans ces trois documents, même ceux réalisés sur mesure, ne permet de détecter et de prévenir d'un défaut d'étanchéité de la protection auditive. Le porteur de la protection croit donc porter un protecteur efficace, alors qu'il ne l'est pas. Ce qui augmente le risque de surexposition au bruit.

### OBJET DE L'INVENTION

La présente invention vise à remédier à tout ou partie de ces inconvénients. La présente invention est divulguée par les revendications: la revendication indépendante 1 divulgue un dispositif portable de protection auditive personnelle et la revendication indépendante 13 un procédé de protection auditive personnelle avec le dispositif divulgué par la revendication 1.

A cet effet, la présente invention vise un dispositif portable de protection auditive personnelle d'un utilisateur, qui comporte :
- deux protecteurs auditifs adaptés, chacun, à atténuer la puissance sonore ambiante perçue par une oreille de l'utilisateur lorsqu'il couvre une oreille interne de l'utilisateur,
- au moins un capteur portable d'une puissance sonore ambiante atténuée par un protecteur dans une oreille interne de l'utilisateur,
- au moins une référence de puissance sonore ambiante non atténuée par un protecteur,
- au moins un moyen de comparaison de la puissance sonore atténuée captée avec la puissance sonore de référence pour détecter un défaut d'étanchéité aux ondes sonores du protecteur auditif et
- au moins un moyen de signalisation configuré pour émettre un signal d'alarme lorsque la comparaison de la puissance sonore captée et de la référence de puissance sonore ambiante dépasse une valeur limite prédéterminée.

Ainsi, le dispositif portable indique à l'utilisateur lorsque l'atténuation par un protecteur auditif est insuffisante du fait d'un défaut d'étanchéité aux ondes sonores du protecteur auditif. Non seulement l'utilisateur est avertit par le signal d'alarme lorsqu'il ne porte pas de protecteur auditif, mais aussi lorsqu'il est mal protégé par le protecteur auditif. Ces caractéristiques permettent d'offrir un dispositif identique pour un grand nombre d'utilisateurs sans avoir besoin de les réaliser sur mesure, c'est-à-dire après prise d'une empreinte de l'oreille interne de l'utilisateur. Le prix de revient du dispositif est ainsi fortement réduit, ce qui permet sa diffusion large et la protection de nombreux travailleurs.

Dans des modes de réalisation, le dispositif objet de la présente invention comporte un capteur de puissance sonore ambiante non atténuée qui fournit la référence de puissance sonore ambiante non atténuée.

Grâce à ces dispositions, la détection du défaut d'étanchéité est permanente.

Dans des modes de réalisation, la référence de puissance sonore ambiante non atténuée est une donnée conservée en mémoire du dispositif et représentative de la puissance sonore moyenne pendant l'activité dans le site considéré.

Ces modes de réalisation évitent d'avoir à prévoir un capteur supplémentaire pour capter la puissance sonore ambiante. Ils présentent donc une complexité et un coût inférieurs.

Le dispositif objet de la présente invention comporte un moyen de détection d'autophonie, le moyen de comparaison de la puissance sonore atténuée captée avec la puissance sonore de référence étant inhibé lorsque le moyen de détection d'autophonie détecte une autophonie.

Grâce à ces dispositions, lorsque les cordes vocales de l'utilisateur sont sollicitées, par exemple lorsqu'il parle ou qu'il chante, on inhibe la détection de défaut d'étanchéité qui serait, sinon, faussée.

Dans des modes de réalisation, le dispositif objet de la présente invention comporte :
- au moins un moyen de comparaison de la puissance sonore captée avec une valeur limite haute prédéterminée représentative d'un niveau de danger pour l'audition de l'utilisateur,
- au moins un moyen de signalisation configuré pour émettre un signal de recommandation de protection auditive complémentaire, lors du dépassement de la valeur limite haute par la puissance sonore ambiante captée.

Si l'utilisateur ne porte pas les protecteurs auditifs la puissance sonore captée est la puissance sonore ambiante, qui est aussi celle présente à l'intérieur de l'oreille interne de l'utilisateur. La protection auditive complémentaire est alors le port des protecteurs auditifs du dispositif. Sinon, soit l'étanchéité sonore des protecteurs est défectueuse, soit l'atténuation des protecteurs est trop faible. Dans le premier cas, la protection auditive complémentaire consiste à mieux positionner ces protecteurs afin de restaurer l'étanchéité nécessaire. Dans le deuxième cas, il s'agit de prévoir une protection additionnelle, par exemple un casque entourant l'oreille externe de l'utilisateur.

La présence d'un capteur portable et d'un moyen de signalisation configuré pour émettre un signal représentatif du dépassement de la valeur limite prédéterminée par la puissance sonore ambiante permet de signaler à l'utilisateur que le port de sa protection auditive personnelle est obligatoire s'il ne veut pas endommager son audition. De plus, la mobilité induite de ce dispositif permet d'établir la dangerosité de l'environnement immédiat de l'utilisateur. Cette prise de conscience par l'utilisateur permet à l'utilisateur de porter ses protecteurs auditifs uniquement quand besoin est. Un tel dispositif permet aussi de responsabiliser l'utilisateur quant à l'utilisation de ses protecteurs auditifs.

Dans des modes de réalisation, le dispositif objet de la présente invention comporte :
- au moins un moyen de comparaison de la puissance sonore captée avec une valeur limite basse prédéterminée représentative d'un niveau de danger de surprotection pour l'utilisateur,
- au moins un moyen de signalisation configuré pour émettre un signal de recommandation de retrait de protection auditive, lorsque la puissance sonore ambiante captée est inférieure à la valeur limite basse.

Ainsi, le dispositif détecte lorsqu'il est inutile de porter les protecteurs auditifs, par exemple parce que l'utilisateur a quitté une zone bruyante et le signale à l'utilisateur afin qu'il évite de conserver les protecteurs auditifs, car ils pourraient empêcher l'utilisateur d'entendre un son utile, par exemple le bruit du passage d'une personne ou d'un véhicule à sa proximité ou un signal d'alarme d'incendie.

Dans des modes de réalisation, ce dispositif objet de l'invention comporte un moyen d'enregistrement des données captées par au moins un capteur de puissance sonore ambiante sur une durée prédéterminée. Ces modes de réalisation ont l'avantage, une fois ces données analysées, de permettre une analyse chronologique de la puissance sonore captée par les capteurs. De plus, ces modes de réalisation permettent d'identifier des valeurs remarquables de puissance sonore captée et d'ajuster les comportements de l'utilisateur en conséquence. En outre, ces modes de réalisation permettent aux employeurs de démontrer qu'ils ont mis à disposition de leurs collaborateurs l'information de dangerosité immédiate de l'ambiance sonore.

Dans des modes de réalisation, le dispositif objet de l'invention comporte un moyen de transmission des données captées par au moins un capteur de puissance sonore ambiante sur une durée prédéterminée. L'avantage de ces modes de réalisation est qu'ils permettent une analyse, en temps réel ou différée selon la présence ou non d'enregistrements par le dispositif, chronologique de la puissance sonore captée par les capteurs. De plus, ces modes de réalisation permettent d'identifier des valeurs remarquables de puissance sonore captée et d'ajuster les comportements de l'utilisateur en conséquence. En outre, ces modes de réalisation permettent aux employeurs de démontrer qu'ils ont mis à disposition de leurs collaborateurs l'information de dangerosité immédiate de l'ambiance sonore.

Dans des modes de réalisation, le moyen de signalisation de recommandation de port des protecteurs auditifs du dispositif comporte un émetteur lumineux. Ces modes de réalisation présentent l'avantage de rendre perceptible le signal de recommandation de port des protecteurs peu importe le niveau sonore environnant.

Dans des modes de réalisation, le moyen de signalisation de recommandation de port des protecteurs auditifs du dispositif objet de l'invention comporte un transducteur électro-acoustique. Ces modes de réalisation ont l'avantage de rendre perceptible le signal de recommandation de port des protecteurs peu importe l'attention visuelle que porte l'utilisateur sur ce moyen de signalisation.

Dans des modes de réalisation, le moyen de signalisation de recommandation de port des protecteurs auditifs du dispositif comporte un émetteur de vibration. L'avantage de ces modes de réalisation est de rendre perceptible le signal de recommandation de port des protecteurs peu importent l'attention visuelle que porte l'utilisateur sur ce moyen de signalisation et l'environnement sonore.

Dans des modes de réalisation, le dispositif objet de l'invention comporte au moins un capteur d'une puissance sonore interne à la protection auditive. Ces modes de réalisation permettent d'évaluer la puissance sonore une fois que les ondes sonores ont passé la protection auditive de l'utilisateur. De telles mesures permettent l'ajustement du comportement de l'utilisateur dans son environnement sonore.

Dans des modes de réalisation, le dispositif comporte un moyen de comparaison de la puissance sonore interne à une valeur limite haute interne prédéterminée et dans lequel le moyen de signalisation est configuré pour émettre un signal de recommandation de port des protecteurs auditifs lors du dépassement de la valeur limite interne par la puissance sonore interne captée. L'avantage de ces modes de réalisation est de permettre d'avertir l'utilisateur si sa protection auditive est mal installée.

Dans des modes de réalisation, le dispositif objet de l'invention comporte un moyen de mesure de l'atténuation de la puissance sonore par comparaison de la puissance sonore interne à la puissance sonore ambiante et dans lequel le moyen de signalisation est configuré pour émettre un signal représentatif du dépassement d'une différence maximale prédéterminée entre les puissances sonores interne et ambiante. L'avantage de ces modes de réalisation est de permettre d'avertir l'utilisateur si sa protection auditive est mal installée ou si l'indice de sa protection est trop faible pour le niveau sonore environnant.

Dans des modes de réalisation, le dispositif objet de la présente invention comporte un moyen de détection d'absence de port des protecteurs auditifs en fonction de la comparaison de la puissance sonore atténuée captée avec la puissance sonore de référence et un moyen de mémorisation de l'absence de port des protecteurs auditifs en correspondance avec une indication de recommandation de port des protecteurs auditifs.

Grâce à ces dispositions, un employeur peut savoir quand l'utilisateur ne s'est pas protégé correctement en présence d'une ambiance sonore nuisible et lui en faire reproche afin qu'ultérieurement il protège son audition lorsque c'est nécessaire.

La présente invention vise également un procédé de protection auditive personnelle d'un utilisateur par utilisation de deux protecteurs auditifs adaptés, chacun, à atténuer la puissance sonore ambiante perçue par une oreille de l'utilisateur lorsqu'il couvre une oreille interne de l'utilisateur, caractérisé en ce qu'il comporte :
- une étape de capteur d'une puissance sonore ambiante atténuée par un protecteur dans une oreille interne de l'utilisateur,
- une étape d'obtention d'au moins une référence de puissance sonore ambiante non atténuée par un protecteur,
- une étape de comparaison de la puissance sonore atténuée captée avec la puissance sonore de référence pour détecter un défaut d'étanchéité aux ondes sonores du protecteur auditif et
- une étape de signalisation pour émettre un signal d'alarme lorsque la comparaison de la puissance sonore captée et de la référence de puissance sonore ambiante dépasse une valeur limite prédéterminée.

Les buts, avantages et caractéristiques de ce dispositif étant similaires à ceux du procédé objet de la présente invention, ils ne sont pas rappelés ici.

### BREVE DESCRIPTION DES FIGURES

D'autres avantages, buts et caractéristiques de l'invention ressortiront de la description qui suit d'au moins un mode de réalisation particulier du dispositif de protection auditive personnelle d'un utilisateur, en regard des dessins annexés, dans lesquels :
- la figure 1 représente, schématiquement, un premier mode particulier de réalisation du dispositif objet de la présente invention,
- la figure 2 représente, schématiquement, un second mode particulier de réalisation du dispositif objet de la présente invention et
- la figure 3 représente un logigramme d'étapes particulier du procédé objet de la présente invention.

### DESCRIPTION D'EXEMPLES DE REALISATION DE L'INVENTION

La présente description est donnée à titre non limitatif.

On note, dès à présent, que les figures ne sont pas à l'échelle.

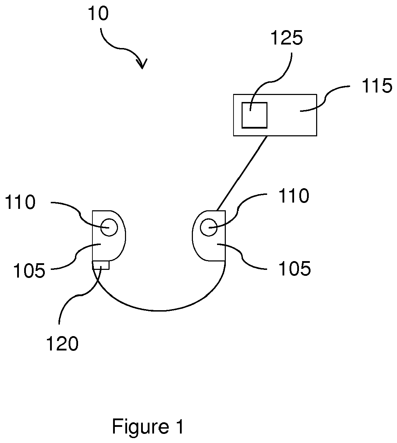

On observe sur la figure 1, le premier mode de réalisation particulier du dispositif 10 objet de la présente invention. Ce dispositif 10 comporte deux protecteurs auditifs 105 adaptés, chacun, à atténuer la puissance sonore ambiante perçue par une oreille de l'utilisateur lorsqu'il couvre une oreille interne de l'utilisateur. Ces protecteurs auditifs 105 portables prennent la forme d'oreillettes destinées à être insérées dans les oreilles internes de l'utilisateur ou sont intégrés à un casque se plaçant sur la tête de l'utilisateur de manière à couvrir ses oreilles dans leur ensemble et, de fait, leur oreille interne également. Ce dispositif 10 comporte également, pour chaque protecteur auditif 105, un capteur 110 portable d'une puissance sonore ambiante atténuée par un protecteur dans une oreille interne de l'utilisateur. Ce capteur 110 portable prend la forme d'un transducteur acoustique-électrique, comme un microphone par exemple et est placé du côté du tympan de l'utilisateur.

Dans cet exemple de réalisation, le capteur portable 110 est directement intégré à un protecteur auditif 105.

Ce dispositif 10 comporte également :
- une référence 125 de puissance sonore ambiante non atténuée par un protecteur,
- un moyen 115 de comparaison de la puissance sonore atténuée captée avec la puissance sonore de référence pour détecter un défaut d'étanchéité aux ondes sonores du protecteur auditif et
- au moins un moyen de signalisation 120 configuré pour émettre un signal d'alarme lorsque la comparaison de la puissance sonore captée et de la référence de puissance sonore ambiante dépasse une valeur limite prédéterminée.

La référence 125 peut être une valeur mémorisée, représentative de l'ambiance sonore moyenne dans le local considéré, pendant les périodes d'activité, ou un transducteur électro-acoustique placé en amont ou à l'extérieur des protecteurs auditifs 105.

Pour effectuer la comparaison, le moyen de comparaison 115 soustrait la puissance sonore atténuée captée, en décibels, à la puissance sonore non atténuée. Connaissant l'atténuation offerte par les protecteurs auditifs lorsque l'étanchéité sonore entre ces protecteurs et l'oreille interne est réalisée, par exemple de 25 dB, le moyen de comparaison 115 détermine si l'étanchéité sonore du port des protecteurs auditifs est suffisante.

Ainsi, le dispositif portable 10 indique à l'utilisateur, par le biais du moyen de signalisation 120, si l'atténuation par un protecteur auditif 105 est insuffisante du fait d'un défaut d'étanchéité aux ondes sonores du protecteur auditif 105. Non seulement l'utilisateur est avertit par le signal d'alarme émis par le moyen de signalisation lorsqu'il ne porte pas de protecteur auditif 105, mais aussi lorsqu'il est mal protégé par le protecteur auditif 105. Ces caractéristiques permettent d'offrir un dispositif 10 identique pour un grand nombre d'utilisateurs sans avoir besoin de les réaliser sur mesure, c'est-à-dire après prise d'une empreinte de l'oreille interne de l'utilisateur. Le prix de revient du dispositif est ainsi fortement réduit, ce qui permet sa diffusion large et la protection de nombreux travailleurs.

Le moyen 120 de signalisation peut prendre la forme d'un buzzer intégré au protecteur auditive ou d'une diode électroluminescente qui clignote en cas de réception par le moyen 115 de comparaison d'un signal représentatif du dépassement de la valeur limite configurée. Cette diode reste éteinte tant que l'atténuation nominale est mesurée par le moyen de comparaison 115. Ce moyen 120 de signalisation est relié par un câble électrique au moyen 115 de comparaison. Dans cet exemple de réalisation, le moyen 120 de signalisation est intégré à un protecteur auditif 105.

Dans des variantes de ce dispositif 10, au moins un élément parmi :
- le capteur 110 portable d'une puissance sonore ambiante,
- le moyen 115 de comparaison de la puissance sonore captée et
- le moyen 120 de signalisation
est déporté du protecteur auditif 105 et est porté n'importe où sur le corps de l'utilisateur, par exemple dans un boîtier déporté qui est porté sur un casque ou sur le col d'un vêtement.

Dans des variantes de ce dispositif 10, au moins deux éléments parmi :
- le capteur 110 portable d'une puissance sonore ambiante,
- le moyen 115 de comparaison de la puissance sonore captée et
- le moyen 120 de signalisation
sont associés dans un unique boitier déporté portable n'importe où sur le corps de l'utilisateur.

Dans des variantes, le dispositif 10 comporte une pluralité de moyens 120 de signalisation portables.

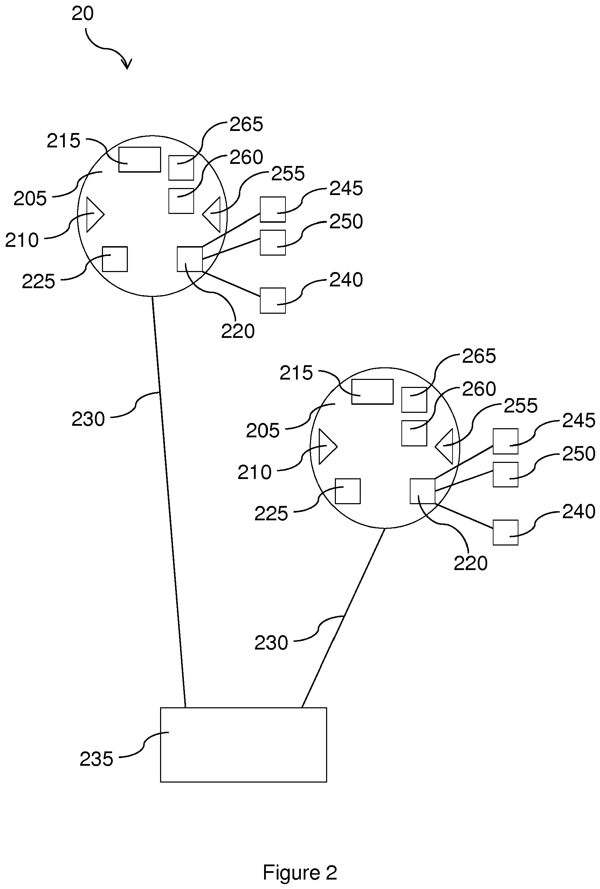

On observe en figure 2, le second mode de réalisation particulier du dispositif 20 objet de la présente invention. Ce dispositif 20 comporte deux protecteurs 205 auditifs adaptés, chacun, à atténuer la puissance sonore ambiante perçue par une oreille de l'utilisateur lorsqu'il couvre une oreille interne de l'utilisateur. Ces protecteurs 205 auditifs prennent la forme d'oreillettes qui se placent dans l'oreille de l'utilisateur de manière à recouvrir de manière étanche l'oreille interne de l'utilisateur.

Chacun des protecteurs 205 auditifs comporte les moyens exposés en regard de la figure 1. Chacun des protecteur auditifs 205 comporte aussi un capteur portable externe d'une puissance sonore ambiante, par exemple un microphone qui fournit la référence 125 de puissance sonore ambiante non atténuée par un protecteur.

Ces protecteurs 205 auditifs comportent également un moyen 210 de comparaison de la puissance sonore captée avec au moins une valeur limite haute prédéterminée représentative d'un niveau de danger pour l'audition de l'utilisateur. Ce moyen 210 prend la forme d'un circuit électronique relié au microphone et configuré pour comparer le signal reçu du microphone avec la valeur limite haute. Ces protecteurs 205 auditifs comportent, de plus, un moyen 220 de signalisation configuré pour émettre un signal de recommandation de port des protecteurs auditifs complémentaires lors du dépassement de la valeur limite haute prédéterminée par la puissance sonore ambiante non atténuée captée.

Préférentiellement, plusieurs valeurs limites hautes sont utilisées, les signaux d'alarme émis étant différents pour représenter le franchissement de chacune des valeurs limites hautes. Ces valeurs limites hautes peuvent, par exemple, correspondre à un des seuils de nocivité qui sont donnés en fonction des durées d'exposition quotidienne au bruit comme le montre le tableau suivant :

| Niveau sonore en dB(A) | Durée d'exposition maximale |
|---|---|
| 80 | 8 heures |
| 83 | 4 heures |
| 86 | 2 heures |
| 89 | une heure |
| 92 | 30 minutes |
| 95 | 15 minutes |

Les moyens 220 de signalisation, situés dans chaque protecteur 205 auditif, comportent un émetteur 240 lumineux comme une diode électroluminescente. Ces moyens 220 de signalisation comportent également un émetteur 250 de vibrations, comme un vibreur. Finalement, les moyens 220 de signalisation comportent un transducteur 245 électro-acoustique comme un buzzer ou un haut-parleur par exemple.

Les protecteurs 205 auditifs comportent également un moyen 225 de comparaison de la puissance sonore atténuée (ou « interne ») à une valeur limite interne haute prédéterminée. Le moyen 220 de signalisation est configuré pour émettre un signal de recommandation de port de protections complémentaires lors du dépassement de la valeur limite interne par la puissance sonore interne captée. Ce moyen 225 prend la forme d'un circuit électronique relié au microphone et configuré pour comparer le signal reçu du microphone avec une valeur seuil prédéterminée. Ces protecteurs 205 auditifs comportent chacun un moyen 265 de mesure de l'atténuation de la puissance sonore par comparaison de la puissance sonore interne à la puissance sonore ambiante et dans lequel le moyen 220 de signalisation est configuré pour émettre un signal représentatif du dépassement d'une différence maximale prédéterminée entre les puissances sonores interne et ambiante.

Le dispositif 20 comporte un moyen 260 d'enregistrement des données captées par le capteur 210 de puissance sonore ambiante sur une durée prédéterminée. Ce moyen 260 prend la forme d'une mémoire déportée dans un boitier 235 portable par l'utilisateur.

Le dispositif 20 comporte un moyen 230 de transmission des données captées par le capteur de puissance sonore ambiante sur une durée prédéterminée. Un tel moyen 230 pouvant être un fil connectant le boitier 235 aux protecteurs 205 auditifs.

Le dispositif 20 comporte aussi un moyen de détection d'autophonie, c'est-à-dire de sons provenant de l'utilisateur, par exemple de ses cordes vocales ou d'un sifflement qu'il émet. A cet effet, le moyen de détection détecte des courtes durées (par exemple inférieures à trois secondes) pendant lesquelles la puissance sonore captée, dans une gamme de longueurs d'ondes (par exemple les sons graves) par le capteur de puissance sonore atténuée est proche de (par exemple à moins de 5 dB), ou supérieure à, la puissance sonore captée par le capteur externe.

Lorsqu'une autophonie est détectée, le moyen de comparaison 115 de la puissance sonore atténuée captée avec la puissance sonore de référence est inhibé.

Préférentiellement, le moyen de détection d'autophonie sépare le signal « voix » du signal « bruit ». A cet effet, il analyse la fréquence et la forme temporelle du signal capté. En effet l'autophonie met en résonance les fréquences graves de la voix. De plus, la forme temporelle d'une voix présente une énergie bien moins importante que celle d'un bruit classique sur une période de l'ordre de quelques secondes. Cela permet de l'isoler en filtrant et en moyennant le signal analysé sur une plus longue période, afin d'extraire l'information de non étanchéité. De la même manière, on élimine également les bruits impulsionnels liés, par exemple, au choc d'un objet (une main, un outil) sur le protecteur, afin d'éviter des signaux d'alarme intempestifs.

Préférentiellement, le dispositif 20 comporte aussi au moins un moyen de comparaison (non représenté) de la puissance sonore captée (atténuée ou non) avec une valeur limite basse prédéterminée représentative d'un niveau de danger de surprotection pour l'utilisateur. Le moyen de signalisation 220 émet un signal de recommandation de retrait de protection auditive, lorsque la puissance sonore ambiante captée est inférieure à la valeur limite basse.

Préférentiellement, le dispositif 20 comporte un moyen de détection d'absence de port des protecteurs auditifs en fonction de la comparaison de la puissance sonore atténuée captée avec la puissance sonore de référence et un moyen de mémorisation de l'absence de port des protecteurs auditifs en correspondance avec une indication de recommandation de port des protecteurs auditifs.

Dans des variantes de ce dispositif 20, le moyen 220 de signalisation est déporté sur le boitier 235 ou est porté, indépendamment du boitier 235, par l'utilisateur.

Dans d'autres variantes de ce dispositif 20, au moins un des éléments comportés par chaque protecteur 205 auditif n'est comporté que par un protecteur 205 auditif.

Dans d'autres variantes de ce dispositif 20, le moyen 230 de communication est composé d'au moins une antenne, disposée sur un protecteur 205 auditif, fonctionnant sur un réseau sans-fil (internet ou téléphonie mobile).

Dans des variantes de ce dispositif 20, les protecteurs 205 auditifs sont reliés entre eux par un fil ou une armature plus rigide.

Finalement, dans des variantes de ce dispositif 20, au moins un des éléments, à l'exception des capteurs 210 d'une puissance sonore interne comportés par chaque protecteur 205 auditif est déporté dans le boitier 235.

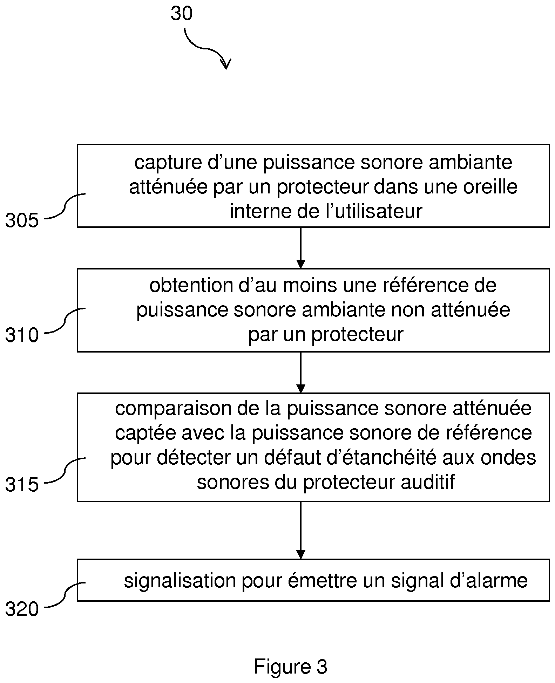

On observe en figure 3, un mode de réalisation particulier du procédé 30 objet de la présente invention. Ce procédé 30 de protection auditive personnelle d'un utilisateur par utilisation de deux protecteurs auditifs adaptés, chacun, à atténuer la puissance sonore ambiante perçue par une oreille de l'utilisateur lorsqu'il couvre une oreille interne de l'utilisateur, comporte :
- une étape 305 de capture d'une puissance sonore ambiante atténuée par un protecteur dans une oreille interne de l'utilisateur,
- une étape 310 d'obtention d'au moins une référence de puissance sonore ambiante non atténuée par un protecteur,
- une étape 315 de comparaison de la puissance sonore atténuée captée avec la puissance sonore de référence pour détecter un défaut d'étanchéité aux ondes sonores du protecteur auditif et
- une étape 320 de signalisation pour émettre un signal d'alarme lorsque la comparaison de la puissance sonore captée et de la référence de puissance sonore ambiante dépasse une valeur limite prédéterminée.

Ce signal prend ici la forme :
- d'une lumière émise par une diode électroluminescente clignotant lorsque la valeur limite prédéterminée est dépassée par la puissance sonore mesurée,
- d'un signal sonore émis par un transducteur électro-acoustique et/ou
- d'une vibration.

La fréquence des alarmes est étudiée pour éviter de gêner l'utilisateur dans son travail. Elles sont plus ou moins espacées suivant la dangerosité de l'exposition.

L'association de la réponse du filtre et de la fréquence du buzzer permet de limiter le niveau du buzzer afin qu'il soit perçu même dans un environnement très bruyant sans ajouter un trop important niveau sonore dans l'oreille.

Pour des utilisateurs n'ayant pas toujours accès à un système de charge, l'autonomie du dispositif est optimisée. On limite au maximum la consommation du dispositif en jouant sur un comparateur « réveillant » le dispositif et une réduction de la consommation des alarmes pour permettre au dispositif de disposer de plusieurs jours, voir semaines, sans recharge. Le dispositif se met automatiquement en veille après être resté deux heures en ne captant qu'une puissance sonore inférieure à une valeur limite basse prédéterminée.

En ce qui concerne l'indication à l'utilisateur qu'il peut enlever ses protections auditives quand le niveau de bruit est très faible, elle évite de placer l'utilisateur en surprotection, ce qui peut s'avérer dangereux. Cette opération place également le dispositif en veille. Un bruit d'une puissance sonore supérieure au premier seuil de dangerosité réveille le dispositif et permet à l'utilisateur d'être prévenu, par l'alarme, qui se déclenche.

Le dispositif peut également comptabiliser les données d'exposition au bruit durant une journée. Chaque fois qu'une des valeurs limites hautes est dépassée le système comptabilise le temps passé au-delà de cette valeur limite haute et lui associe une valeur plus ou moins importante suivant le niveau de seuil. L'addition de toutes ces valeurs peut être comparée à une dose limite d'exposition au bruit. On peut également récupérer ces données par un système câblé ou sans fil.

Le dispositif réinitialise automatiquement son compteur entre deux journées en détectant une période de N heures durant laquelle le capteur de bruit ambiant détecte une puissance sonore ambiante inférieure à une valeur limite « de silence » prédéfinie.

Dans des modes de réalisation, le dispositif comporte un moyen de commande du niveau d'atténuation manuellement, sans enlever cette protection, et donc déplacer le micro de l'oreille, pour obtenir une mesure plus précise tout en gardant le confort de l'utilisateur. Par exemple, la modulation du niveau d'atténuation met en oeuvre des pièces mécaniques pour déplacer différents filtres. Par exemple, on utilise un barillet avec trois filtres d'atténuation et un canal ouvert pour permettre au son de passer et un totalement obstrué dans le cas de très forts niveaux sonores. L'utilisateur peut faire tourner le barillet jusqu'à ce que l'alarme s'arrête, ou l'ouvrir lorsque l'alarme de surprotection se déclenche. Dans des variantes, on prévoit un système automatique pour faire tourner le barillet en fonction du niveau sonore.

Le dispositif peut être rechargé de différentes manières :
- câblé avec un chargeur ou
- sur un dock permettant également de récupérer les données.

Dans ce cas, dans le domaine de l'industrie, les docks peuvent être montés en rack et nominatifs. Les docks peuvent permettre, en plus de la recharge et de la récupération des données, de nettoyer les protecteurs pour une plus grande durée de vie et une meilleure hygiène. Cela est possible, par exemple, par des solutions
- de séchage (problème de la transpiration dans les conduits menant à l'électronique),
- de pulvérisation de produits et/ou
- de nettoyage aux rayonnements ultraviolets (UVs),

Le boîtier comporte une diode électroluminescente pour indiquer l'état de charge de sa source d'énergie électrique. Pour être rechargé, le boîtier est équipé d'une connectique. Dans des variantes, on met en oeuvre une recharge par induction sur une surface plane, ce qui permet d'étanchéifier le boîtier. Dans ce cas, la récupération des données peut être effectuée par la mise en oeuvre d'une technologie sans fil (Bluetooth, marque déposée, ou NFC, acronyme de « Near Field Communication » pour communication en champ proche, par exemple).

Dans des variantes, on prévoit une source d'énergie renouvelable comme le rechargement solaire en plaçant des panneaux solaires sur le casque ou directement sur la partie exposée du protecteur.

Dans des variantes, le boîtier permet de transmettre, en plus de l'analyse de l'étanchéité et du niveau/dose de bruit, des appel téléphoniques, radio ou de filtrer le bruit pour transmettre la parole dans une conversation en face à face. Un signal venant d'un transducteur électro-acoustique permet de faire passer des messages plus complexes et également de transmettre la parole, voire de la musique.

## Revendications

1. Dispositif (10, 20) portable de protection auditive personnelle d'un utilisateur, **caractérisé en ce qu'**il comporte :
- deux protecteurs (105, 205) auditifs adaptés, chacun, à atténuer la puissance sonore ambiante perçue par une oreille de l'utilisateur lorsqu'il couvre une oreille interne de l'utilisateur,
- au moins un capteur (110) portable d'une puissance sonore ambiante atténuée par un protecteur dans une oreille interne de l'utilisateur,
- au moins une référence (125) de puissance sonore ambiante non atténuée par un protecteur,
- au moins un moyen (115) de comparaison de la puissance sonore atténuée captée avec la puissance sonore de référence pour détecter un défaut d'étanchéité aux ondes sonores du protecteur auditif,
- au moins un moyen (120) de signalisation configuré pour émettre un signal d'alarme lorsque la comparaison de la puissance sonore captée et de la référence de puissance sonore ambiante dépasse une valeur limite prédéterminée et
- un moyen de détection d'autophonie, le moyen de comparaison de la puissance sonore atténuée captée avec la puissance sonore de référence étant inhibé lorsque le moyen de détection d'autophonie détecte une autophonie.

2. Dispositif selon la revendication 1, qui comporte un capteur de puissance sonore ambiante non atténuée qui fournit la référence de puissance sonore ambiante non atténuée.

3. Dispositif selon la revendication 2, qui comporte :
- au moins un moyen (215) de comparaison de la puissance sonore captée avec une valeur limite haute prédéterminée représentative d'un niveau de danger pour l'audition de l'utilisateur,
- au moins un moyen (220) de signalisation configuré pour émettre un signal de recommandation de protection auditive complémentaire, lors du dépassement de la valeur limite haute par la puissance sonore ambiante captée.

4. Dispositif selon l'une des revendications 2 ou 3, qui comporte :
- au moins un moyen de comparaison de la puissance sonore captée avec une valeur limite basse prédéterminée représentative d'un niveau de danger de surprotection pour l'utilisateur,
- au moins un moyen de signalisation configuré pour émettre un signal de recommandation de retrait de protection auditive, lorsque la puissance sonore ambiante captée est inférieure à la valeur limite basse.

5. Dispositif selon la revendication 1, dans lequel la référence de puissance sonore ambiante non atténuée est une donnée conservée en mémoire du dispositif et représentative de la puissance sonore moyenne pendant l'activité dans le site considéré.

6. Dispositif (20) selon l'une des revendications 1 à 5, qui comporte un moyen (260) d'enregistrement des données captées par au moins un capteur (210) de puissance sonore ambiante sur une durée prédéterminée.

7. Dispositif (20) selon l'une des revendications 1 à 6, qui comporte un moyen (230) de transmission des données captées par au moins un capteur (210) de puissance sonore ambiante sur une durée prédéterminée.

8. Dispositif (20) selon l'une des revendications 1 à 7, dans lequel le moyen (220) de signalisation comporte un émetteur (240) lumineux.

9. Dispositif (20) selon l'une des revendications 1 à 8, dans lequel le moyen (220) de signalisation comporte un transducteur (245) électro-acoustique.

10. Dispositif (20) selon l'une des revendications 1 à 9, dans lequel le moyen (220) de signalisation comporte un émetteur (250) de vibration.

11. Dispositif (20) selon l'une des revendications 1 à 10, qui comporte un moyen de (225) comparaison de la puissance sonore interne à une valeur limite haute interne prédéterminée et dans lequel le moyen (220) de signalisation est configuré pour émettre un signal de recommandation de port des protecteurs (105) auditifs lors du dépassement de la valeur limite interne par la puissance sonore interne captée.

12. Dispositif (20) selon l'une des revendications 1 à 11, qui comporte un moyen de détection d'absence de port des protecteurs auditifs en fonction de la comparaison de la puissance sonore atténuée captée avec la puissance sonore de référence et un moyen de mémorisation de l'absence de port des protecteurs auditifs en correspondance avec une indication de recommandation de port des protecteurs auditifs.

13. Procédé de protection auditive personnelle d'un utilisateur par utilisation de deux protecteurs (105, 205) auditifs adaptés, chacun, à atténuer la puissance sonore ambiante perçue par une oreille de l'utilisateur lorsqu'il couvre une oreille interne de l'utilisateur, **caractérisé en ce qu'**il comporte :
- une étape (305) de capture d'une puissance sonore ambiante atténuée par un protecteur dans une oreille interne de l'utilisateur,
- une étape (310) d'obtention d'au moins une référence de puissance sonore ambiante non atténuée par un protecteur,
- une étape (315) de comparaison de la puissance sonore atténuée captée avec la puissance sonore de référence pour détecter un défaut d'étanchéité aux ondes sonores du protecteur auditif,
- une étape (320) de signalisation pour émettre un signal d'alarme lorsque la comparaison de la puissance sonore captée et de la référence de puissance sonore ambiante dépasse une valeur limite prédéterminée et
- une étape de détection d'autophonie, la comparaison de la puissance sonore atténuée captée avec la puissance sonore de référence étant inhibée lorsqu'une autophonie est détectée au cours de l'étape de détection d'une autophonie.

## Patentansprüche

1. Tragbare persönliche Gehörschutzvorrichtung für einen Benutzer, **dadurch gekennzeichnet, dass** sie Folgendes umfasst:
- zwei Gehörschützer (105, 205), die jeweils ausgeführt sind, um die Umgebungsschallleistung, die durch ein Ohr des Benutzers wahrgenommen wird, abzuschwächen, wenn sie ein Innenohr des Benutzers abdecken,
- zumindest einen tragbaren Sensor (110) für eine Umgebungsschallleistung, die durch einen Schützer im Innenohr des Benutzers abgeschwächt wird,
- zumindest eine Referenz (125) für eine nicht durch einen Schützer abgeschwächte Umgebungsschallleistung,
- zumindest ein Mittel (115) zum Vergleich der empfangenen abgeschwächten Schallleistung mit der Referenzschallleistung zum Erfassen eines Fehlers bei der Undurchlässigkeit gegenüber den Schallwellen des Gehörschützers,
- zumindest ein Mittel (120) zum Signalisieren, das konfiguriert ist, um ein Alarmsignal zu senden, wenn der Vergleich der empfangenen Schallleistung und der Referenz für die Umgebungsschalleistung einen vorbestimmten Grenzwert übersteigt, und
- ein Mittel zur Erfassung von Autophonie, wobei das Mittel zum Vergleich der empfangenen abgeschwächten Schallleistung mit der Referenzschallleistung unterdrückt wird, wenn das Mittel zur Autophonie-Erfassung eine Autophonie erfasst.

2. Vorrichtung nach Anspruch 1, die einen Sensor für eine nicht abgeschwächte Umgebungsschalleistung umfasst, der die Referenz für die nicht abgeschwächte Umgebungsschalleistung beisteuert.

3. Vorrichtung nach Anspruch 2, Folgendes umfassend:
- zumindest ein Mittel (215) zum Vergleich der empfangenen Schallleistung mit einem vorbestimmten oberen Grenzwert, der repräsentativ für eine Gefahrenstufe für das Gehör des Benutzers ist,
- zumindest ein Mittel (220) zum Signalisieren, das konfiguriert ist, um bei Überschreitung des oberen Grenzwerts durch die empfangene Umgebungsschalleistung ein Signal zur Empfehlung eines ergänzenden Gehörschutzes zu senden.

4. Vorrichtung nach einem der Ansprüche 2 oder 3, Folgendes umfassend:
- zumindest ein Mittel zum Vergleich der empfangenen Schallleistung mit einem vorbestimmten unteren Grenzwert, der repräsentativ für eine Gefahrenstufe für einen übermäßigen Schutz für den Benutzer ist,
- zumindest ein Mittel zum Signalisieren, das konfiguriert ist, um ein Signal zur Empfehlung der Abnahme des Gehörschutzes zu senden, wenn die empfangene Umgebungsschalleistung geringer als der untere Grenzwert ist.

5. Vorrichtung nach Anspruch 1, wobei die Referenz für die nicht abgeschwächte Umgebungsschalleistung ein Datum ist, dass im Speicher der Vorrichtung aufbewahrt wird, und repräsentativ für die durchschnittliche Schallleistung während der Tätigkeit am betrachteten Ort ist.

6. Vorrichtung (20) nach einem der Ansprüche 1 bis 5, die ein Mittel (260) zum Speichern der durch zumindest einen Sensor (210) für eine Umgebungsschalleistung über eine vorbestimmte Dauer empfangenen Daten umfasst.

7. Vorrichtung (20) nach einem der Ansprüche 1 bis 6, die ein Mittel (230) zum Übertragen der durch zumindest einen Sensor (210) für eine Umgebungsschalleistung über eine vorbestimmte Dauer empfangenen Daten umfasst.

8. Vorrichtung (20) nach einem der Ansprüche 1 bis 7, wobei das Mittel (220) zum Signalisieren einen Lichtemitter (240) umfasst.

9. Vorrichtung (20) nach einem der Ansprüche 1 bis 8, wobei das Mittel (220) zum Signalisieren einen elektroakustischen Wandler (245) umfasst.

10. Vorrichtung (20) nach einem der Ansprüche 1 bis 9, wobei das Mittel (220) zum Signalisieren einen Vibrationssender (250) umfasst.

11. Vorrichtung (20) nach einem der Ansprüche 1 bis 10, die ein Mittel (225) zum Vergleich der internen Schallleistung mit einem vorbestimmten oberen internen Grenzwert umfasst, und wobei das Mittel (220) zum Signalisieren konfiguriert ist, um bei Überschreitung des internen Grenzwerts durch die empfangene interne Schallleistung ein Signal zur Empfehlung zum Tragen der Gehörschützer (105) zu senden.

12. Vorrichtung (20) nach einem der Ansprüche 1 bis 11, die ein Mittel zum Erfassen des Nicht-Tragens der Gehörschützer in Abhängigkeit vom Vergleich der empfangenen abgeschwächten Schallleistung mit der Referenzschallleistung umfasst, und ein Mittel zum Speichern des Nicht-Tragens der Gehörschützer entsprechend einem Hinweis zur Empfehlung zum Tragen der Gehörschützer.

13. Verfahren zum persönlichen Schutz des Gehörs eines Benutzers durch die Verwendung von zwei Gehörschützen (105, 205), die jeweils ausgeführt sind, um die Umgebungsschalleistung, die durch ein Ohr des Benutzers wahrgenommen wird, abzuschwächen, wenn sie ein Innenohr des Benutzers abdecken, **dadurch gekennzeichnet, dass** es Folgendes umfasst:
- einen Schritt (305) zum Erfassen einer Umgebungsschalleistung, die durch einen Schützer im Innenohr des Benutzers abgeschwächt wird,
- einen Schritt (310) für den Erhalt zumindest einer Referenz für eine nicht durch einen Schützer abgeschwächte Umgebungsschalleistung,
- einen Schritt (315) zum Vergleich der empfangenen abgeschwächten Schallleistung mit der Referenzschallleistung zum Erfassen eines Fehlers bei der Undurchlässigkeit gegenüber den Schallwellen des Gehörschützers,
- einen Schritt (320) zum Signalisieren, um ein Alarmsignal zu senden, wenn der Vergleich der empfangenen Schallleistung und der Referenz für die Umgebungsschalleistung einen vorbestimmten Grenzwert übersteigt, und
- einen Schritt zur Erfassung von Autophonie, wobei der Vergleich der empfangenen abgeschwächten Schallleistung mit der Referenzschallleistung unterdrückt wird, wenn im Laufe des Schrittes zur Erfassung von Autophonie eine Autophonie erfasst wird.

## Claims

1. Portable device (10, 20) for the personal auditory protection of a user, **characterized in that** it comprises:
- two auditory protectors (105, 205), each designed to attenuate the ambient sound power perceived by an ear of the user when it covers an inner ear of the user;
- at least one portable sensor (110) for sensing an ambient sound power attenuated by a protector in an inner ear of the user;
- at least one ambient sound power reference (125) not attenuated by a protector;
- at least one means (115) for comparing the sensed attenuated sound power with the reference sound power in order to detect a sound wave sealing defect of the auditory protector;
- at least one signaling means (120) configured to emit a warning signal when the comparison of the sensed sound power and the ambient sound power reference exceeds a predefined threshold value; and
- an autophony detection means, the means for comparing the sensed attenuated sound power with the reference sound power being inhibited when the autophony detection means detects an autophony.

2. Device according to claim 1, that comprises a non-attenuated ambient sound power sensor that provides the non-attenuated ambient sound power reference.

3. Device according to claim 2, that comprises:
- at least one means (215) for comparing the sensed sound power with a predefined upper threshold value representative of a danger level for the user's hearing;
- at least one signaling means (220) configured to emit a signal recommending additional auditory protection when the sensed ambient sound power exceeds the upper threshold value.

4. Device according to one of claims 2 or 3, that comprises
- at least one means for comparing the sensed sound power with a predefined lower threshold value representative of an overprotection danger level for the user;
- at least one signaling means configured to emit a signal recommending the removal of the auditory protection when the sensed ambient sound power is below the lower threshold value.

5. Device according to claim 1, wherein the non-attenuated ambient sound power reference is an item of data stored in the device's memory and representative of the average sound power during activity on the site in question.

6. Device (20) according to one of claims 1 to 5, that comprises a means (260) for recording the data captured by at least one ambient sound power sensor (210) over a predefined period of time.

7. Device (20) according to one of claims 1 to 6, that comprises a means (230) for transmitting the data captured by at least one ambient sound power sensor (210) over a predefined period of time.

8. Device (20) according to one of claims 1 to 7, wherein the signaling means (220) comprises a light emitter (240).

9. Device (20) according to one of claims 1 to 8, wherein the signaling means (220) comprises an electro-acoustic transducer (245).

10. Device (20) according to one of claims 1 to 9, wherein the signaling means (220) comprises a vibration emitter (250).

11. Device (20) according to one of claims 1 to 10, that comprises a means (225) for comparing the internal sound power with a predefined internal upper threshold value, and wherein the signaling means (220) is configured to emit a signal recommending the wearing of auditory protectors (105) when the sensed internal sound power exceeds the internal threshold value.

12. Device (20) according to one of claims 1 to 11, that comprises a means for detecting the non-wearing of auditory protectors as a function of the comparison of the sensed attenuated sound power with the reference sound power, and a means for storing the non-wearing of auditory protectors corresponding to an indication of recommending the wearing of auditory protectors.

13. Method for the personal auditory protection of a user by the use of two auditory protectors (105, 205), each designed to attenuate the ambient sound power perceived by an ear of the user when it covers an inner ear of the user, **characterized in that** it comprises:
- a step (305) of sensing an ambient sound power attenuated by a protector in an inner ear of the user;
- a step (310) of obtaining at least one ambient sound power reference not attenuated by a protector;
- a step (315) for comparing the sensed attenuated sound power with the reference sound power in order to detect a sound wave sealing defect of the auditory protector;
- a signaling step (320) to emit a warning signal when the comparison of the sensed sound power and the ambient sound power reference exceeds a predefined threshold value; and
- an autophony detection step, the comparison of the sensed attenuated sound power with the reference sound power being inhibited when an autophony is detected during the autophony detection step.
